# FASCICULE DE BREVET EUROPEEN

(11) **EP 4 479 101 B1**
(45) Date de publication et mention de la délivrance du brevet: **04.03.2026**
(21) Numéro de dépôt: 23708265.6
(22) Date de dépôt: 17.02.2023
(51) Int. Cl.: A61L 24/04, A61L 15/08, A61L 15/58

(54) **COMPOSITION PHOTOPOLYMERISABLE POUR ADHESIF POUR TISSUS BIOLOGIQUES**
PHOTOPOLYMERISIERBARE ZUSAMMENSETZUNG FÜR BIOLOGISCHE GEWEBEKLEBSTOFFE
PHOTOPOLYMERISABLE COMPOSITION FOR ADHESIVE FOR BIOLOGICAL TISSUE

(30) Priorité: 18.02.2022 FR 2201481
(43) Date de publication de la demande: 25.12.2024
(73) Titulaire: Cohesives, 21000 Dijon (FR)
(72) Inventeur: PERRIN, Bertrand, 21000 DIJON (FR); GOUTAY, Natacha, 21000 DIJON (FR)
(74) Mandataire: Icosa
(86) Numéro de dépôt international: PCT/FR2023/050229
(87) Numéro de publication internationale: WO 2023/156749

(56) Documents cités:
- WO-A1-2016/185153
- US-A1- 2020 172 769

## Description

### DOMAINE DE L'INVENTION

L'invention concerne une composition polymérisable, destinée à être utilisée en tant qu'adhésif pour tissus biologiques, en particulier pour des tissus biologiques non minéralisés. La composition de l'invention permet notamment, après polymérisation, l'adhésion de tissus biologiques entre eux, l'adhésion d'un matériau, d'une colle ou d'une substance à un tissu biologique, la formation d'un produit d'étanchéification chirurgical ou la formation d'un pansement cutané.

### ÉTAT DE LA TECHNIQUE

Les adhésifs des pansements classiques, notamment cutanés, supportent mal l'humidité, ce qui entraine leur décollement. L'humidité peut provenir des exsudats de la plaie, de la transpiration ou des activités quotidiennes.

Les adhésifs médicaux présentent aussi un intérêt pour remplacer ou faciliter les sutures et remplacer les pansements. L'application des adhésifs médicaux se fait la plupart du temps directement sur le tissu, sans préparation de la surface de collage. Les adhésifs médicaux actuellement disponibles ont des propriétés adhésives parfois insuffisantes et/ou une faible stabilité dans le temps.

Par ailleurs, des adhésifs médicaux peuvent être utilisés dans un certain nombre de techniques chirurgicales. Toutefois, l'efficacité des colles chirurgicales actuellement disponibles est controversée.

Il existe donc un besoin pour améliorer les propriétés des adhésifs médicaux.

Les adhésifs médicaux peuvent se présenter sous la forme de compositions polymérisables. La polymérisation peut se faire par exemple sous l'action de l'humidité des tissus sur lesquels la composition est appliquée, ou par un amorçage chimique ou physique externe, tel que par exemple sous l'effet d'un rayonnement UV ou visible.

Afin d'améliorer les propriétés des adhésifs médicaux, des compositions comprenant un monomère photopolymérisable et ayant de faibles viscosités ont été proposées (WO2016/185153). Ces compositions pénètrent plus facilement dans les tissus, ce qui permet d'obtenir un collage de meilleure qualité grâce à un ancrage de l'adhésif dans le tissu après polymérisation. Dans certaines indications, ces collages peuvent nécessiter d'être associés à un adhésif supplémentaire pour en améliorer les propriétés. De plus ces compositions sont difficilement utilisables directement par le grand public.

La présente invention se propose de fournir un nouveau type d' adhésif médical permettant de résoudre ces différents problèmes. Les compositions selon l'invention comprennent un monomère polymérisable de type acrylate, une résine photopolymérisable et un photoamorceur.

Les compositions selon l'invention permettent d'obtenir une adhésion adaptée, aussi bien pour des applications chirurgicales, que pour une adhésion cutanée grand public.

Les concentrations des constituants de la composition de l'invention permettent un amorçage rapide et une polymérisation en quelques dizaines de secondes, sans occasionner de brûlure des tissus grâce à une exothermie maitrisée.

De plus, la présence combinée d'une résine photopolymérisable, avec le photoamorceur et les monomères polymérisables utilisés, apporte au collage obtenu des propriétés de flexibilité et conformabilité adaptées aux tissus biologiques mous, une bonne stabilité dans le temps sous l'action des liquides physiologiques (e.g. sang, exsudats, transpiration), empêche l'accumulation de transpiration ou d'exsudats et confère une bonne résistance à l'eau.

### RÉSUMÉ

La présente invention concerne donc une composition polymérisable, destinée à être utilisée en tant qu' adhésif pour tissus biologiques non minéralisés, comprenant :
- de 5 % à 60 % en masse, par rapport à la masse totale de la composition, d'un monomère polymérisable comprenant une fonction acrylate, méthacrylate, acrylamide ou méthacrylamide et ne comprenant pas de fonction phosphate ou phosphonate ;
- de 0,1 % à 5 % en masse d'un photoamorceur ; et
- une résine photopolymérisable ;
ladite composition ne comprenant pas de monomère comprenant une fonction phosphate ou phosphonate d'une part et une fonction acrylate, méthacrylate, acrylamide ou méthacrylamide d'autre part.

Selon un mode de réalisation, le monomère polymérisable est choisi parmi : acide acrylique (AA), acrylate de tert-butyl (tBuA), méthacrylate de 2-hydroxyéthyl (HEMA), acide méthacrylique (MA), acrylate de lauryle (LA), méthacrylate de lauryle (LMA), méthacrylate de 2-éthoxyéthyle (2EEMA), acrylate de di(éthylène glycol) éthyle éther (DEGEA), acrylate de 2-phényloxyéthyle (2PEA), méthacrylate de 2-éthylhexyle (2EHMA), acrylate de n-butyle (nBuA), acrylate d'isobornyle (IBOA), méthacrylate d'isobornyle (IBOMA), cyclic triméthylolpropane formal acrylate (CTFA), méthacrylate de 3,3,5 triméthyl cyclohexanol, méthacrylate de tert-butyl (tBuMA), acrylate de méthyle (MeA), méthacrylate de méthyle (MMA), acrylate de 2-éthylhexyle (2EHA), acrylate de 2-(diméthylamino)éthyle (DAEA), sel potassique d'acrylate de 3-sulfopropyle (SAPS), acide 3,3-diméthylacrylique (DAA), acide crotonique (CA), méthacrylate de triéthylène glycol méthyle éther (TEGMEMA), méthacrylate de 2-phényloxyéthyle (2PEMA), acrylate de 2-hydroxyéthyle (HEA), méthacrylate de 3-(triméthoxysilyl)propyle, acrylate d'éthyle (EtA), méthacrylate de cyclohexyle, acrylate de 3-hydroxypropyle, acrylate d'alpha-bromoéthyle, acrylate d'alpha-chloroéthyle, méthacrylate de chlorométhyle, méthacrylate de 2-bromoéthyle, méthacrylate de 2-naphtyle, acrylate de paratolyle, méthacrylate de parachlorophényle, acrylate de metabromophényle, acrylate de 2,4,6-tribromophényle, méthacrylate de parachlorobenzyle, méthacrylate de metaméthoxybenzyle, acrylate de paraéthylbenzyle, diméthacrylate de 1,6-hexanediol, diacrylate de neopentylglycol, diméthacrylate de thiodiéthylène-glycol, diacrylate de bisphénol A éthoxyle, diméthacrylate de bisphénol A éthoxyle, triacrylate de pentaérythritol, triacrylate de glycéryle, pentaacrylate de dipentaérythritol, triacrylate de triméthylolpropane, triméthacrylate de l'isocyanurate de tris(2-hydroxyéthyle), triacrylate de triméthylolpropane polyoxyéthylène, acrylate d'uréthane, methacrylate d'uréthane, sulfure de bis(4-methacryloylthiophényle), acrylate d'éthylèneglycol, acrylate de polyéthylèneglycol, méthacrylate d'éthylèneglycol, méthacrylate de polyéthylèneglycol, diacrylate d'éthylèneglycol, diacrylate de polyéthylèneglycol, diméthacrylate d'éthylèneglycol, diméthacrylate de polyéthylèneglycol, et leurs mélanges.

Selon un mode de réalisation, le monomère polymérisable est choisi parmi : acide acrylique (AA), acrylate de tert-butyl (tBuA), méthacrylate de 2-hydroxyéthyl (HEMA), acide méthacrylique (MA), acrylate de lauryle (LA), méthacrylate de lauryle (LMA), méthacrylate de 2-éthoxyéthyle (2EEMA), acrylate de di(éthylène glycol) éthyle éther (DEGEA), acrylate de 2-phényloxyéthyle (2PEA), méthacrylate de 2-éthylhexyle (2EHMA), acrylate de n-butyle (nBuA), acrylate d'isobornyle (IBOA), méthacrylate d'isobornyle (IBOMA), cyclic triméthylolpropane formal acrylate (CTFA), méthacrylate de 3,3,5 triméthyl cyclohexanol, méthacrylate de tert-butyl (tBuMA), acrylate de méthyle (MeA), méthacrylate de méthyle (MMA), acrylate de 2-éthylhexyle (2EHA), acrylate de 2-(diméthylamino)éthyle (DAEA), sel potassique d'acrylate de 3-sulfopropyle (SAPS), acide 3,3-diméthylacrylique (DAA), acide crotonique (CA), méthacrylate de triéthylène glycol méthyle éther (TEGMEMA), méthacrylate de 2-phényloxyéthyle (2PEMA), acrylate de 2-hydroxyéthyle (HEA), méthacrylate de 3-(triméthoxysilyl)propyle, et leurs mélanges.

Selon un mode de réalisation, le monomère polymérisable est à une concentration allant de 10 % à 40 % en masse par rapport à la masse totale de la composition.

Selon un mode de réalisation, le photoamorceur est choisi parmi l'oxyde de 2,4,6-triméthylbenzoyl-phénylphosphinate (TPO-L), la camphorquinone, la 4,4'-bis(diéthylamino)benzophénone, la 4,4'-bis(diéthylamino)benzophénone associée à la N-phénylglycine (NPG), à l'éthyl-4- (diméthylamino)benzoate (EDB), à la N-diisopropyléthylamine (DIPEAN) ou au 4-(diméthylamino)benzonitrile (DMABN), l'oxyde de biacylphosphine (BAPO), le bis(.eta.5-2,4-cylcopentadièn-1-yl)-bis(2,6-difluoro-3-(1H-pyrrol-1-yl)-phényl) titanium (Irgacure 784), la 1-[4-(2-hydroxyéthoxy)-phényl]-2-hydroxy-2-méthyl-1-propane-1-one (Irgacure 2959), l'oxyde de 2,4,6-triméthylbenzoyldiphénylphosphine (TPO), la 2,2-diméthoxyphényl-2-acétophénone (DMPA), et leurs mélange.

Selon un mode de réalisation, le photoamorceur est à une concentration allant de 0,1 % à 2 % en masse par rapport à la masse totale de la composition.

Selon un mode de réalisation, la résine photopolymérisable est choisie parmi : les résines uréthane acrylate, les résines uréthane méthacrylate, les résines époxy acrylate, et leurs mélanges ; de préférence la résine photopolymérisable est choisie parmi les résines uréthane acrylate, les résines uréthane méthacrylate, les résines époxy acrylate difonctionnelle et leurs mélanges; plus préférentiellement la résine photopolymérisable est choisie parmi : une résine uréthane acrylate, une résine uréthane méthacrylate et leurs mélanges; encore plus préférentiellement, la résine photopolymérisable est sélectionnée parmi les résines uréthanes acrylates aliphatiques, les résines uréthanes acrylates hydrophobes, les résines uréthanes acrylates aromatiques, les résines polyéthers uréthanes acrylates, les résines uréthanes méthacrylates aliphatiques, les résines uréthanes méthacrylates hydrophobes, les résines uréthanes méthacrylates aromatiques, les résines polyéthers uréthanes méthacrylates, et leurs mélanges.

Selon un mode de réalisation, la résine photopolymérisable est à une concentration allant de 10% à 94,9 % en masse par rapport à la masse totale de la composition.

L'invention concerne également une composition selon l'invention, pour son utilisation pour l'adhésion de tissus biologiques non minéralisés entre eux ; pour l'adhésion d'un matériau à un tissu biologique non minéralisé ; pour l'adhésion d'une colle ou d'une substance à la surface d'un tissu biologique non minéralisé ; en tant qu'étanchéificateur chirurgical sur un tissu biologique non minéralisé ; pour obturer ou colmater les orifices crées par une suture par fil ou par agrafe ou par une résection tissulaire dans un tissu biologique non minéralisé ; pour obturer un orifice, une incision ou une déchirure dans un tissu biologique non minéralisé ; en tant qu'hémostatique pour arrêter un saignement sur un tissu biologique non minéralisé , en tant que pansement sur un tissu biologique non minéralisé pour recouvrir et protéger une plaie ; pour renforcer un tissu biologique non minéralisé ; pour prévenir la formation de lésions sur un tissu biologique non minéralisé ; pour fixer et stabiliser un tissu biologique non minéralisé ; et/ou pour le traitement des lésions cutanées.

Selon un mode de réalisation, la composition pour son utilisation selon l'invention, comprend la mise en contact de la composition avec le tissu biologique non minéralisé à traiter, de préférence par étalement ; et la photopolymérisation de ladite composition.

La présente invention concerne aussi un procédé de préparation d'une composition polymérisable destinée à être utilisée en tant qu'adhésif pour tissus biologiques non minéralisés, comprenant une étape de mélange :
- d'au moins un monomère polymérisable comprenant une fonction acrylate, méthacrylate, acrylamide ou méthacrylamide et ne comprenant pas de fonction phosphate ou phosphonate ;
- d'au moins un photoamorceur ; et
- d'au moins une résine photopolymérisable ;
ledit mélange ne comprenant pas de monomère comprenant une fonction phosphate ou phosphonate d'une part et une fonction acrylate, méthacrylate, acrylamide ou méthacrylamide d'autre part.

Selon un mode de réalisation, la masse du monomère polymérisable varie de 5 % à 60% en masse, par rapport à la masse totale dudit mélange ; et la masse du photoamorceur varie de 0,1% à 5% en masse, par rapport à la masse totale dudit mélange.

La présente invention concerne aussi un kit comprenant la composition de l'invention telle que décrit précédemment.

Selon un mode de réalisation, le kit comprend en outre une source de rayonnement.

### DÉFINITIONS

Dans la présente invention, les termes ci-dessous sont définis de la manière suivante :
- « **Composition polymérisable** » fait référence à une composition comprenant un ou plusieurs composants pouvant être polymérisés pour former un polymère.
- « **Monomère polymérisable** » fait référence à un monomère dont la polymérisation peut être initiée par un amorceur physique ou chimique.
- « **Photoamorceur** » fait référence à un composé qui, ajouté à un monomère, permet d'amorcer la photopolymérisation en créant des espèces réactives (e.g. radicaux libres, cations, anions) sous l'effet d'un rayonnement (UV ou visible).
- « **Résine polymérisable** » fait référence à un oligomère réactif de haut poids moléculaire (généralement supérieur à 450 g/mol, de préférence supérieur à 1000 g/mol) pouvant être polymérisé. Dans la présente invention, la résine polymérisable est une résine photopolymérisable, c'est-à-dire dont la polymérisation peut être initiée sous l'effet d'un rayonnement.
- « **Tissus biologiques** » fait référence à un ensemble de cellules semblables et de même origine, regroupées en amas, réseau ou faisceau (fibre). Un tissu biologique forme un ensemble fonctionnel, c'est-à-dire que ses cellules concourent à une même fonction. Les tissus biologiques sont assemblés entre eux pour former des organes. Dans la présente invention, l'expression « tissus biologique » englobe également les organes d'une manière générale.
- « **Tissus biologiques non minéralisés** » fait référence à des tissus biologiques à l'exclusion des tissus osseux, des os et des dents. Les tissus biologiques non minéralisés sont en général des tissus mous.
- Par « **pharmaceutiquement acceptable** » se référant à une substance ou à une composition, on entend que les substances ou la composition sont compatibles entre elles et/ou non délétères pour le sujet, de préférence un être humain, auquel la substance ou la composition est administrée. En particulier, elles ne produisent pas de réaction indésirable, allergique ou autre lors de l'administration au sujet. Pour l'administration humaine, les compositions doivent répondre aux normes de stérilité, de pyrogénicité, de sécurité générale et de pureté requises par les organismes de réglementation, tels que la *Food and Drug Administration (FDA)* ou l'Agence européenne des médicaments (EMA).- « **Sujet** » concerne un animal à sang chaud, de préférence un mammifère, plus préférentiellement un humain. De préférence, le sujet est un patient, c'est-à-dire un sujet qui attend de recevoir, ou qui reçoit des soins médicaux, ou qui fait/va faire l'objet d'un acte médical.
- **« De [valeur inférieure] à [valeur supérieure]** » et d'autres expressions similaires définissent une plage numérique qui inclut (c'est-à-dire englobe) à la fois la valeur supérieure et la valeur inférieure. De plus, toute plage ainsi définie dans la présente demande doit être interprétée comme incluant une divulgation explicite de la plage plus étroite correspondante décrite par l'expression « **entre [valeur inférieure] et [valeur supérieure]** », dans laquelle à la fois la valeur supérieure et la valeur inférieure sont exclues de la plage numérique (c'est-à-dire non englobées).
- Le terme « **comprend** » entend signifier que la composition selon l'invention inclut les éléments cités. Ce terme englobe également les compositions comprenant uniquement les éléments cités à l'exclusion de tout autre (composition « **consistant en** » les éléments cités).

### DESCRIPTION DÉTAILLÉE

### Composition polymérisable

La présente invention concerne donc une composition polymérisable, destinée à être utilisée en tant qu'adhésif pour tissus biologiques, en particulier pour des tissus biologiques non minéralisés, permettant notamment l'adhésion de tissus biologiques entre eux, l'adhésion d'un matériau à un tissu biologique, la formation d'un produit d'étanchéification chirurgical, ou la formation d'un pansement cutané.

L'invention concerne notamment des compositions polymérisables destinées à être utilisées en tant qu'adhésif pour tissus biologiques, en particulier pour des tissus biologiques non minéralisés, comprenant :
- au moins un monomère polymérisable comprenant une fonction acrylate, méthacrylate, acrylamide ou méthacrylamide et ne comprenant pas de fonction phosphate ou phosphonate ;
- au moins un amorceur de polymérisation, de préférence un photoamorceur ; et
- au moins une résine photopolymérisable.

La polymérisation de la composition de l'invention permet de former l'adhésif visé et d'obtenir des propriétés d'adhésion pour le système résultant.

Par « **adhésif pour tissus biologiques** », il est fait référence à un adhésif destiné à un usage sur des tissus biologiques, en particulier un usage médical sur des tissus biologiques. Les constituants d'un tel adhésif et de la composition polymérisable permettant de le former doivent donc être par nature compatibles avec les tissus biologiques et notamment les usages médicaux. En particulier, ils ne doivent pas provoquer d'effet délétère (i.e. réaction indésirable, allergique ou autre) sur les tissus biologiques concernés.

La composition de l'invention est notamment destinée à être utilisée pour des tissus biologiques non minéralisés. Cela suppose qu'une fois appliquée et polymérisée, ladite composition doit permettre d'obtenir d'un adhésif ayant des propriétés physicochimiques compatibles avec des tissus biologiques non minéralisés, c'est-à-dire des tissus mous. En particulier, les propriétés suivantes doivent être obtenues :
- une bonne flexibilité de l'adhésif, adaptée à un usage sur tissu biologique mou, afin de protéger le tissu de l'extérieur tout en assurant un confort de port (absorption des chocs, résistance et résilience face aux grandes déformations) ;
- une bonne stabilité dans le temps sous l'action des liquides physiologiques (e.g. sang, exsudats, transpiration), afin notamment de maintenir les propriétés d'adhésion en milieu humide et de ne pas entrainer de déformation au contact des liquides physiologiques pour maintenir la protection mécanique et l'adhésion au tissu biologique ; et
- empêcher l'accumulation de transpiration ou d'exsudats.

Dans le cadre de la présente invention, lorsque les concentrations des différents composants de la composition selon l'invention sont indiquées en pourcentage il s'agit du pourcentage en masse dudit composant par rapport à la masse totale de ladite composition.

### Monomères polymérisables

La composition de l'invention comprend un monomère polymérisable comprenant une fonction acrylate, méthacrylate, acrylamide ou méthacrylamide et ne comprenant pas de fonction phosphate ou phosphonate. Dans la présente invention, ce type de monomère pourra être désigné par l'expression « **monomère polymérisable non-phosphaté** ».

Selon un mode de réalisation, le monomère polymérisable non-phosphaté est sélectionné parmi les monomères suivants : acide acrylique (AA), acrylate de tert-butyl (tBuA), méthacrylate de 2-hydroxyéthyl (HEMA), acide méthacrylique (MA), acrylate de lauryle (LA), méthacrylate de lauryle (LMA), méthacrylate de 2-éthoxyéthyle (2EEMA), acrylate de di(éthylène glycol) éthyle éther (DEGEA), acrylate de 2-phényloxyéthyle (2PEA), méthacrylate de 2-éthylhexyle (2EHMA), acrylate de n-butyle (nBuA), acrylate d'isobornyle (IBOA), méthacrylate d'isobornyle (IBOMA), cyclic triméthylolpropane formal acrylate (CTFA), méthacrylate de 3,3,5 triméthyl cyclohexanol, méthacrylate de tert-butyl (tBuMA), acrylate de méthyle (MeA), méthacrylate de méthyle (MMA), acrylate de 2-éthylhexyle (2EHA), acrylate de 2-(diméthylamino)éthyle (DAEA), sel potassique d'acrylate de 3-sulfopropyle (SAPS), acide 3,3-diméthylacrylique (DAA), acide crotonique (CA), méthacrylate de triéthylène glycol méthyle éther (TEGMEMA), méthacrylate de 2-phényloxyéthyle (2PEMA), acrylate de 2-hydroxyéthyle (HEA), méthacrylate de 3-(triméthoxysilyl)propyle, acrylate d'éthyle (EtA), méthacrylate de cyclohexyle, acrylate de 3-hydroxypropyle, acrylate d'alpha-bromoéthyle, acrylate d'alpha-chloroéthyle, méthacrylate de chlorométhyle, méthacrylate de 2-bromoéthyle, méthacrylate de 2-naphtyle, acrylate de paratolyle, méthacrylate de parachlorophényle, acrylate de metabromophényle, acrylate de 2,4,6-tribromophényle, méthacrylate de parachlorobenzyle, méthacrylate de metaméthoxybenzyle, acrylate de paraéthylbenzyle, diméthacrylate de 1,6-hexanediol, diacrylate de neopentylglycol, diméthacrylate de thiodiéthylène-glycol, diacrylate de bisphénol A éthoxyle, diméthacrylate de bisphénol A éthoxyle, triacrylate de pentaérythritol, triacrylate de glycéryle, pentaacrylate de dipentaérythritol, triacrylate de triméthylolpropane, triméthacrylate de l'isocyanurate de tris(2-hydroxyéthyle), triacrylate de triméthylolpropane polyoxyéthylène, acrylate d'uréthane, méthacrylate d'uréthane, sulfure de bis(4-methacryloylthiophényle), acrylate d'éthylèneglycol, acrylate de polyéthylèneglycol, méthacrylate d'éthylèneglycol, méthacrylate de polyéthylèneglycol, diacrylate d'éthylèneglycol, diacrylate de polyéthylèneglycol, diméthacrylate d'éthylèneglycol, diméthacrylate de polyéthylèneglycol, et leurs mélanges.

Selon un mode de réalisation, le monomère polymérisable non-phosphaté est sélectionné parmi les monomères suivants : acide acrylique (AA), acrylate de *tert*-butyl (tBuA), méthacrylate de 2-hydroxyéthyl (HEMA), acide méthacrylique (MA), acrylate de lauryle (LA), méthacrylate de lauryle (LMA), méthacrylate de 2-éthoxyéthyle (2EEMA), acrylate de di(éthylène glycol) éthyle éther (DEGEA), acrylate de 2-phényloxyéthyle (2PEA), méthacrylate de 2-éthylhexyle (2EHMA), acrylate de *n*-butyle (nBuA), acrylate d'isobornyle (IBOA), méthacrylate d'isobornyle (IBOMA), acrylate de triméthylolpropane cyclique formal (CTFA), méthacrylate de 3,3,5 triméthyl cyclohexanol (TMCHMA), méthacrylate de tert-butyl (tBuMA), acrylate de méthyle (MeA), méthacrylate de méthyle (MMA), acrylate de 2-éthylhexyle (2EHA), acrylate de 2-(diméthylamino)éthyle (DAEA), sel potassique d'acrylate de 3-sulfopropyle (SAPS), acide 3,3-diméthylacrylique (DAA), acide crotonique (CA), méthacrylate de triéthylène glycol méthyle éther (TEGMEMA), méthacrylate de 2-phényloxyéthyle (2PEMA), acrylate de 2-hydroxyéthyle (HEA), méthacrylate de 3-(triméthoxysilyl)propyle, et leurs mélanges.

Selon un mode de réalisation, le monomère polymérisable non-phosphaté est sélectionné parmi les monomères suivants : acrylate de *tert*-butyle (tBuA), méthacrylate d'hydroxyéthyle (HEMA), acide acrylique (AA), acrylate de lauryle (LA), acrylate d'isobornyle (IBOA), méthacrylate d'isobornyle (IBOMA), méthacrylate de 3,3,5 triméthyl cyclohexanol (TMCHMA), et leurs mélanges.

Selon un mode de réalisation, le monomère polymérisable non-phosphaté est un mélange de deux ou plus des monomères listés ci-dessus. En particulier, le monomère polymérisable non-phosphaté est un mélange d'acide acrylique et d'un autre monomère listé ci-dessus.

Selon un mode de réalisation, le monomère polymérisable non-phosphaté a une masse molaire allant de 50 g.mol⁻¹ à 500 g.mol⁻¹, de préférence de 70 g.mol⁻¹ à 400 g.mol⁻¹.

Selon un mode de réalisation, le monomère polymérisable non-phosphaté est présent dans la composition de l'invention à une concentration allant de 5 % à 60 % en masse par rapport à la mase totale de la composition, de préférence de 5 % à 50 % en masse, de préférence de 5 % à 40 % en masse. Selon un mode de réalisation, le monomère polymérisable non-phosphaté est présent dans la composition de l'invention à une concentration allant de 10 % à 40 % en masse de préférence de 20 % à 40 % en masse, plus préférentiellement de 25 % à 35 % en masse. Selon un autre mode de réalisation, le monomère polymérisable non-phosphaté est présent dans la composition de l'invention à une concentration allant de 5 % à 30 % en masse, plus préférentiellement de 10 % à 20 % en masse.

La polymérisation du monomère polymérisable non-phosphaté présent dans la composition de l'invention peut être initiée par un amorceur physique ou chimique. Selon un mode de réalisation préféré, la polymérisation est initiée sous l'effet d'un rayonnement, de préférence un rayonnement ayant une longueur d'onde allant de 300 nm à 520 nm, de préférence de 350 nm à 520 nm. Les monomères utilisés dans la composition de l'invention présentent l'avantage de polymériser rapidement même à des gammes d'irradiance faible à modérée, de préférence allant de 5 mW/cm² à 250 mW/cm², de préférence de 10 mW/cm² à 100 mW/cm², de préférence de 10 mW/cm² à 50 mW/cm². Ces gammes d'irradiance faible à modérée sont particulièrement adaptées à une utilisation sur des tissus biologiques car elles n'émettent pas d'UV C. De plus, ces gammes d'irradiance faible à modérée présentent l'avantage d'être accessible avec des sources de rayonnement légères, par opposition aux sources de rayonnement industrielles qui sont encombrantes et ont de fortes irradiances (>10 W/cm²).

Selon un mode de réalisation, la composition selon l'invention ne comprend pas de monomère comprenant d'une part une fonction phosphate ou phosphonate et d'autre part une fonction méthacrylate, acrylate, acrylamide ou méthacrylamide. Dans la présente invention, ce type de monomère pourra être désigné par l'expression « **monomère polymérisable phosphaté** ». Selon un mode de réalisation, la composition selon l'invention ne comprend pas un monomère polymérisable sélectionné parmi le glycérol diméthacrylate phosphate, l'éthylène glycol méthacrylate phosphate, le polyéthylène glycol méthacrylate phosphate, le méthacryloyloxy décyl hydrogène phosphate, le méthacryloyloxy l'éthyloxy hydrogène phosphate, le glycérol monométhacrylate phosphate, le triéthylène glycol monométhacrylate phosphate, le méthacryloyloxy propyle phosphate, le méthacryloyloxy hexyle phosphate, l'acide phosphonique aminoéthyle méthacrylé, bis(glycéryl diméthacrylate) phosphate et leurs mélanges. Selon un mode de réalisation, la composition selon l'invention ne comprend pas un monomère polymérisable phosphaté sélectionné parmi CAS [14206-25-8], [14235-57-5], [86242-61-7], [932019-41-6], [1980781-17-6], [60161-88-8], [87243-97-8], [1980048-95-0], [918802-80-9], [63411-25-6], [1980064-07-0], [22432-83-3], [1980781-08-5], [252210-28-9], [1114567-37-1], [80730-17-2], [518991-74-7], [87243-96-7], [1980062-84-7], [518991-75-8], [252210-30-3], [22432-84-4], [727415-30-7], [727415-31-8], [784139-89-5] ou [1194231-98-5] et leurs mélanges. Selon un mode de réalisation, la composition selon l'invention ne comprend pas un monomère polymérisable phosphaté de formule I tel que décrit dans WO2020/169681, en particulier le 10-MDP (C₁₄H₂₇O₆P, numéro CAS [85590-00-7]) ou le MEP(C₁₂H₁₉O₈P, numéro CAS [32435-46-4]).

Selon un mode de réalisation, la composition selon l'invention ne comprend pas de monomère dont la polymérisation peut être initiée au seul contact de l'eau, de l'humidité des tissus biologiques et/ou de l'humidité ambiante. Selon un mode de réalisation, la composition selon l'invention ne comprend pas de monomère de la famille des cyanoacrylates.

### Résines photopolymérisables

La composition de l'invention comprend une résine photopolymérisable. Avantageusement, la présence d'une résine photopolymérisable dans la composition de l'invention confère à l'adhésif obtenu après polymérisation des propriétés de flexibilité optimales pour les applications visées, notamment pour l'adhésion des tissus biologiques non minéralisés, tout en limitant l'exothermie de la réaction de polymérisation de la composition à un niveau acceptable. Une exothermie acceptable correspond à une température lors de la polymérisation de la composition n'entrainant pas de brûlure des tissus sur lesquels la composition est polymérisée, ni de sensation de brûlure. De préférence, l'exothermie est maitrisée pour assurer une température de polymérisation inférieure à 60°C.

Par ailleurs, la présence de la résine photopolymérisable dans la composition de l'invention contribue à conférer à l'adhésif obtenu après polymérisation une bonne stabilité dans le temps sous l'action des liquides physiologiques et une bonne résistance à l'eau, et à empêcher l'accumulation de transpiration ou d'exsudats.

Selon un mode de réalisation, la résine photopolymérisable utilisée dans la composition de l'invention est une résine qui forme un matériau flexible après polymérisation.

Selon un mode de réalisation, la résine photopolymérisable est choisie parmi les résines uréthane acrylate, les résines uréthane méthacrylate, les résines époxy acrylate, et leurs mélanges.

Selon un mode de réalisation, la résine photopolymérisable est une résine uréthane acrylate ou une résine uréthane méthacrylate. Selon un mode de réalisation particulier, la résine photopolymérisable est une résine uréthane acrylate.

Selon un mode de réalisation, la résine uréthane est sélectionnée parmi les résines uréthanes acrylates aliphatiques, les résines uréthanes acrylates hydrophobes, les résines uréthanes acrylates aromatiques, les résines polyéthers uréthanes acrylates, les résines uréthanes méthacrylates aliphatiques, les résines uréthanes méthacrylates hydrophobes, les résines uréthanes méthacrylates aromatiques, les résines polyéthers uréthanes méthacrylates, et leurs mélanges. Selon un mode de réalisation, la résine uréthane est sélectionnée parmi les résines uréthanes acrylates aliphatiques, les résines uréthanes acrylates hydrophobes, les résines uréthanes acrylates aromatiques, les résines polyéthers uréthanes acrylates, et leurs mélanges.

Selon un mode de réalisation, la résine uréthane acrylate aliphatique est sélectionnée parmi Allnex Ebecryl 9907^{®}, Allnex Ebecryl 230^{®}, Allnex Ebecryl 250^{®}, Allnex Ebecryl 8315^{®} (tétraacrylate), Allnex Ebecryl 4491^{®}, Allnex Ebecryl 1271^{®}, et leurs mélanges. Selon un mode de réalisation, la résine uréthane acrylate aromatique est le Allnex Ebecryl 210^{®}. Selon un mode de réalisation, la résine uréthane acrylate hydrophobe est le Dymax Bomar BRC-843S^{®}. Selon un mode de réalisation, la résine polyéther uréthane acrylate est le Dymax Bomar BR-3641AJ^{®}.

Selon un mode de réalisation, la résine photopolymérisable est sélectionnée parmi Allnex Ebecryl 9907^{®}, Allnex Ebecryl 230^{®}, Allnex Ebecryl 250^{®}, Allnex Ebecryl 8315^{®} (tétraacrylate), Allnex Ebecryl 4491^{®}, Allnex Ebecryl 1271^{®}, Allnex Ebecryl 210^{®}, Dymax Bomar BRC-843S^{®}, Dymax Bomar BR-3641AJ^{®}, et leurs mélanges.

Selon un mode de réalisation particulier, la résine photopolymérisable est la Allnex Ebecryl 9907^{®}.

Selon un mode de réalisation, la résine photopolymérisable est une résine époxy acrylate ; de préférence une résine époxy acrylate difonctionnelle ; plus préférentiellement la résine époxy acrylate difonctionnelle est choisie parmi : les résines bisphénol A époxy diacrylate, telles que par exemple Allnex Ebecryl 3708^{®}, les résines acrylates d'huile de soja époxydé, telle que par exemple Rahn Genomer 2312^{®}, et leurs mélanges.

Selon un autre mode de réalisation, la résine photopolymérisable est une résine époxy acrylate difonctionnelle, en particulier en résine bisphénol A époxy diacrylate, telle que par exemple Allnex Ebecryl 3708^{®}.

Selon un mode de réalisation, la composition de l'invention comprend au moins 10 % en masse par rapport à la masse totale de la composition, de résine photopolymérisable. Selon un mode de réalisation, la composition de l'invention comprend de 10 % à 94,9 % en masse, de résine photopolymérisable, de préférence de 50 % à 94,9 %. Selon un mode de réalisation, la composition de l'invention comprend de 65% à 94,75%, en masse, de résine photopolymérisable. Selon un mode de réalisation, la composition de l'invention comprend 65%, 68%, 69%, 79%, 89% et 94, 75% en masse de résine photopolymérisable.

La polymérisation de la résine photopolymérisable peut être initiée sous l'effet d'un rayonnement. De manière préférée, ledit rayonnement a une longueur d'onde allant de 300 nm à 520 nm, de préférence de 350 nm à 520 nm. De manière préférée ledit rayonnement UV a une puissance d'irradiance allant de 5 mW/cm² à 250 mW/cm², de préférence de 10 mW/cm² à 100 mW/cm², de préférence de 10 mW/cm² à 50 mW/cm².

### Photoamorceurs

La composition de l'invention comprend un amorceur de polymérisation, de préférence un photoamorceur.

Selon un mode de réalisation, le photoamorceur est capable d'induire une polymérisation sous l'effet d'un rayonnement ayant une longueur d'onde allant de 300 nm à 520 nm, de préférence de 350 nm à 520 nm.

Selon un mode de réalisation, le photoamorceur choisi parmi : l'oxyde de 2,4,6-triméthylbenzoyl-phénylphosphinate (TPO-L), la camphorquinone, la 4,4'-bis(diéthylamino)benzophénone, la 4,4'-bis(diéthylamino)benzophénone associée à la N-phénylglycine (NPG), à l'éthyl-4- (diméthylamino)benzoate (EDB), à la N-diisopropyléthylamine (DIPEAN) ou au 4-(diméthylamino)benzonitrile (DMABN), l'oxyde de biacylphosphine (BAPO), le bis(.eta.5-2,4-cylcopentadièn-1-yl)-bis(2,6-difluoro-3-(1H-pyrrol-1-yl)-phényl) titanium (Irgacure 784), la 1-[4-(2-hydroxyéthoxy)-phényl]-2-hydroxy-2-méthyl-1-propane-1-one (Irgacure 2959), l'oxyde de 2,4,6-triméthylbenzoyldiphénylphosphine (TPO), la 2,2-diméthoxyphényl-2-acétophénone (DMPA), et leurs mélanges.

Selon un mode de réalisation particulier, le photoamorceur est le TPO-L.

Selon un mode de réalisation, le photoamorceur est présent dans la composition de l'invention à une concentration allant de 0,1 % à 5 % en masse, par rapport à la masse totale de la composition, de préférence de 0,1 % à 2 % en masse, de préférence de 0,25 % à 2 % en masse, plus préférentiellement de 0,5 % à 2 % en masse.

De manière inattendue, le photoamorceur joue un rôle sur les propriétés d'adhésion après polymérisation, qu'on peut faire varier en fonction de la quantité et du type de photoamorceur utilisé.

L'utilisation d'un amorceur de polymérisation thermique ou redox n'est pas exclue du cadre de la présente invention. Parmi les amorceurs redox utilisables, on peut notamment citer le couple dibenzoyle peroxide/amine (triméthylaniline).

### Compositions particulières

Selon un mode de réalisation particulier, la composition de l'invention comprend :
- de 5 % à 30 % en masse, par rapport à la masse totale de la composition, d'un monomère polymérisable non-phosphaté ;
- de 0,25 % à 5 % en masse d'un photoamorceur ; et
- de 65 % à 94,75 % en masse d'une résine photopolymérisable.

Selon un mode de réalisation particulier, la composition de l'invention comprend :
- de 10 % à 30 % en masse, par rapport à la masse totale de la composition, d'un monomère polymérisable non-phosphaté sélectionné parmi acrylate de tert-butyle (tBuA), méthacrylate d'hydroxyéthyle (HEMA), acide acrylique (AA), acrylate de lauryle (LA), acrylate d'isobornyle (IBOA), méthacrylate d'isobornyle (IBOMA), et méthacrylate de 3,3,5 triméthyl cyclohexanol (TMCHMA) ;
- de 0,25 % à 5 % en masse de TPO-L ; et
- de 65 % à 94,75 % en masse de résine uréthane acrylate aliphatique telle que Allnex Ebecryl 9907^{®}.

Selon un mode de réalisation particulier, la composition de l'invention comprend :
- de 10 % à 30 % en masse, par rapport à la masse totale de la composition, d'un monomère polymérisable non-phosphaté sélectionné parmi acrylate de *tert*-butyle (tBuA), méthacrylate d'hydroxyéthyle (HEMA), acide acrylique (AA), acrylate de lauryle (LA), acrylate d'isobornyle (IBOA), méthacrylate d'isobornyle (IBOMA), et méthacrylate de 3,3,5 triméthyl cyclohexanol (TMCHMA) ;
- de 0,25 % à 5 % en masse de TPO-L ; et
- de 65 % à 94,75 %, de préférence de 65% à 89,75%, en masse d'une résine photopolymérisable choisie parmi les résines uréthane acrylate aliphatique telle que Allnex Ebecryl 9907^{®} ; les résines époxy acrylate difonctionnelle telles que la résine époxy acrylate Allnex Ebecryl 3708^{®} (correspondant à une résine diacrylate d'époxy bisphénol A modifié) ou la résine époxy acrylate Rahn Genomer 2312^{®} (correspondant à une résine acrylate d'huile de soja époxydé), et leurs mélanges.

Selon un mode de réalisation, la composition de l'invention comprend en outre un véhicule pharmaceutiquement acceptable. Selon un autre mode de réalisation, la composition de l'invention est dépourvue de solvant.

### Propriétés des compositions

Selon un mode de réalisation, la composition de l'invention est sous forme liquide.

Selon un mode de réalisation, la composition de l'invention présente une viscosité inférieure à 120 Pa.s à 20°C, de préférence inférieure à 107 Pa.s à 20°C, de préférence inférieure à 55 Pa.s à 20°C. La viscosité de la composition peut notamment être mesurée par un viscosimètre rotatif, un viscosimètre cône/plan, un viscosimètre à chute de bille, un viscosimètre à capillaire en verre, un viscosimètre à coupe d'écoulement ou par extrusion sous pression, de préférence selon la norme NF EN 12092.

L'ajustement de la viscosité de la composition permet notamment de contrôler l'épaisseur de la couche de composition déposée sur le tissu biologique avant sa polymérisation, en fonction de l'utilisation visée pour l'adhésif formé après polymérisation. Par exemple, une couche fine, d'une épaisseur inférieure ou égale à 0,5 mm, est préférée pour l'adhésion d'un matériau à un tissu biologique ou pour l'adhésion d'une colle ou d'une substance à la surface d'un tissu biologique. Une couche plus épaisse, d'une épaisseur supérieure ou égale à 1 mm, est préférée pour la formation d'un pansement sur un tissu biologique pour recouvrir et protéger une plaie.

Selon un mode de réalisation, la composition selon l'invention n'est pas un hydrogel.

### Kit

La présente invention concerne également un kit, ou ensemble de parties, comprenant une composition selon l'invention et une source de rayonnement.

De préférence la source de rayonnement de l'ensemble de parties peut émettre un rayonnement adapté pour polymériser et/ou aider la polymérisation et/ou accélérer la polymérisation des constituants de la composition.

Dans le cadre de la présente invention, le terme « **source de rayonnement** » fait référence à tout moyen artificiel apte à produire un rayonnement de longueur d'onde allant de 300 nm à 520 nm, de préférence de 350 nm à 520 nm. De manière préférée ledit rayonnement UV a une puissance d'irradiance allant de 5 mW/cm² à 250 mW/cm², de préférence de 10 mW/cm² à 100 mW/cm², de préférence de 10 mW/cm² à 50 mW/cm². Avantageusement, ces gammes d'irradiance peuvent être obtenue par des sources de rayonnement légères, adaptés à un usage grand public, par opposition aux sources de rayonnement industrielles qui sont encombrantes et ont de fortes irradiances (>10 W/cm²). Selon un mode de réalisation, la source de rayonnement est une source LED UV.

### Procédé de préparation d'une composition polymérisable

L'invention concerne également un procédé de préparation d'une composition polymérisable selon l'invention. En particulier, l'invention porte sur un procédé de préparation d'une composition polymérisable destinée à être utilisée en tant qu'adhésif pour tissus biologiques non minéralisés, ledit procédé comprenant ou étant constitué par une étape de mélange :
- d'au moins un monomère polymérisable comprenant une fonction acrylate, méthacrylate, acrylamide ou méthacrylamide, et ne comprenant pas de fonction phosphate ou phosphonate tel que décrit précédemment ;
- d'au moins un photoamorceur tel que décrit précédemment; et
- d'au moins une résine photopolymérisable tel que décrit précédemment;

ledit mélange ne comprenant pas de monomère comprenant une fonction phosphate ou phosphonate d'une part et une fonction acrylate, méthacrylate, acrylamide ou méthacrylamide d'autre part.

Selon un mode de réalisation, la masse du monomère polymérisable dans le mélange représente de 5% à 60% de la masse totale dudit mélange.

Selon un mode de réalisation, la masse du photoamorceur dans le mélange représente de 0,1% à 5% de la masse totale dudit mélange.

### Utilisation des compositions polymérisables

L'invention concerne également l'utilisation des compositions polymérisables de l'invention en tant qu'adhésif pour tissus biologiques, en particulier pour des tissus biologiques non minéralisés.

En particulier, les compositions polymérisables de l'invention peuvent être utilisées sur des tissus biologiques, en particulier sur des tissus biologiques non minéralisés, comme adhésif chirurgical, produit d'étanchéification chirurgical, ou pansement cutané.

Plus particulièrement, la présente invention concerne l'utilisation des compositions polymérisables de l'invention, sur des tissus biologiques, en particulier des tissus biologiques non minéralisés:
- pour l'adhésion des tissus biologiques entre eux,
- pour l'adhésion d'un matériau à un tissu biologique,
- pour l'adhésion d'une colle ou d'une substance à la surface d'un tissu biologique,
- en tant qu'étanchéificateur chirurgical,
- pour obturer ou colmater les orifices créés par une suture par fil ou par agrafe ou par une résection tissulaire (hémostase, aérostase, lymphostase par exemple),
- pour obturer un orifice, une incision ou une déchirure dans un tissu biologique,
- en tant qu'hémostatique pour arrêter un saignement, seul ou en complément de techniques conventionnelles d'hémostase comme la suture, la compression ou l'électrocoagulation,
- en tant que pansement sur un tissu biologique pour recouvrir et protéger une plaie,
- pour renforcer un tissu biologique,
- pour fixer et stabiliser un tissu biologique,
- pour le traitement des lésions cutanées.

L'invention concerne donc une composition polymérisable selon l'invention pour son utilisation sur des tissus biologiques, en particulier sur des tissus biologiques non minéralisés, comme adhésif chirurgical, produit d'étanchéification chirurgical, ou pansement cutané.

La présente invention concerne également une composition polymérisable selon l'invention pour son utilisation en tant qu'adhésif chirurgical pour l'adhésion de tissus biologiques entre eux, pour l'adhésion d'un matériau à un tissu biologique, pour l'adhésion d'une colle ou d'une substance à la surface d'un tissu biologique, en tant qu'étanchéificateur chirurgical, pour obturer ou colmater les orifices crées par une suture par fil ou par agrafe ou par une résection tissulaire, pour obturer un orifice, une incision ou une déchirure dans un tissu biologique, en tant qu'hémostatique pour arrêter un saignement, en tant que pansement sur un tissu biologique pour recouvrir et protéger une plaie, pour renforcer un tissu biologique, pour fixer et stabiliser un tissu biologique, pour le traitement des lésions cutanées. Les tissus biologiques sont de préférence des tissus biologiques non minéralisés.

La présente invention concerne également une composition selon l'invention pour son utilisation en tant qu'adhésif chirurgical pour l'adhésion de tissus biologiques non minéralisés entre eux, par exemple pour réaliser une suture, notamment une suture de plaie cutanée.

La présente invention concerne également une composition selon l'invention pour son utilisation en tant qu'adhésif chirurgical pour l'adhésion d'un matériau à un tissu biologique non minéralisé, par exemple pour faire adhérer un pansement ou un champ opératoire à la peau.

La présente invention concerne également une composition selon l'invention pour utilisation en tant qu'adhésif chirurgical pour l'adhésion d'une colle ou d'une substance à la surface d'un tissu biologique non minéralisé, par exemple pour renforcer les propriétés d'une colle chirurgicale.

La présente invention concerne également une composition selon l'invention pour son utilisation en tant qu'étanchéificateur chirurgical, notamment pour obturer ou colmater les orifices crées par une suture par fil ou par agrafe ou par une résection tissulaire, dans un tissu biologique non minéralisé.

La présente invention concerne également une composition selon l'invention pour son utilisation en tant qu'étanchéificateur chirurgical pour obturer un orifice, une incision ou une déchirure dans un tissu biologique non minéralisé.

La présente invention concerne également une composition selon l'invention pour son utilisation en tant qu'hémostatique pour arrêter un saignement.

La présente invention concerne également une composition selon l'invention pour son utilisation pour former un pansement sur un tissu biologique non minéralisé pour recouvrir et protéger une plaie.

La présente invention concerne également une composition selon l'invention pour son utilisation en tant qu'adhésif chirurgical pour renforcer un tissu biologique non minéralisé.

La présente invention concerne également une composition selon l'invention pour son utilisation en tant qu' adhésif chirurgical pour prévenir la formation de lésions sur un tissu, notamment un tissu cutané ; par exemple pour prévenir la formation d'ampoules.

La présente invention concerne également une composition selon l'invention pour son utilisation en tant qu'adhésif chirurgical pour fixer et stabiliser un tissu biologique non minéralisé.

La présente invention concerne également une composition selon l'invention pour son utilisation pour le traitement de lésions cutanées.

Il a notamment été mis en évidence que les compositions selon l'invention présentaient des capacités adhésives et une innocuité supérieure aux compositions de l'art antérieur. Ces compositions sont donc particulièrement adaptées pour une utilisation cutanée comme pansement pour les blessures et/ou pour le traitement de toutes lésions cutanées.

### Procédé de mise en œuvre des compositions de l'invention

La présente invention concerne également un procédé de mise en œuvre de la composition polymérisable de l'invention, comprenant les étapes suivantes :
- (i) mise en contact du tissu biologique à traiter avec une composition selon l'invention ;
- (ii) optionnellement laisser pénétrer la composition dans ledit tissu ; et
- (iii) induire la polymérisation de ladite composition.

Selon un mode de réalisation, dans l'étape (i), la mise en contact du tissu biologique à traiter avec la composition peut se faire par étalement, pulvérisation ou par mise en contact à l'aide d'un matériau préenduit tel qu'un patch préenduit. Selon un mode de réalisation, la mise en contact se fait par étalement.

Selon un mode de réalisation, l'étape (i) vise à enduire totalement ou en partie le tissu biologique à traiter.

Selon un mode de réalisation, l'étape (ii) est optionnelle. Le temps de pénétration de la composition dans le tissu avant sa polymérisation peut aller de 0 à 5 minutes.

Selon un mode de réalisation préféré, l'étape (iii) est réalisée à l'aide d'un rayonnement UV ou de lumière visible. Les caractéristiques du rayonnement mis en œuvre, notamment sa puissance et sa longueur d'onde, sont adaptées aux constituants de la composition, notamment à la nature du monomère polymérisable et à la nature de l'amorceur de polymérisation. Selon un mode de réalisation, le rayonnement utilisé pour induire la polymérisation a une longueur d'onde allant de 300 nm à 520 nm, de préférence de 350 nm à 520 nm. Selon un mode de réalisation, le rayonnement utilisé pour induire la polymérisation a une puissance d'irradiance allant de 5 mW/cm² à 250 mW/cm², de préférence de 10 mW/cm² à 100 mW/cm², de préférence de 10 mW/cm² à 50 mW/cm².

Selon un mode de réalisation, la composition de l'invention donne après polymérisation un système biocompatible.

Les compositions selon l'invention permettent d'obtenir après polymérisation une excellente adhésion, aussi bien pour des applications chirurgicales que pour un niveau d'adhésion cutanée adapté à une application par le grand public.

Les concentrations des constituants de la composition de l'invention permettent un amorçage rapide et une polymérisation en quelques dizaines de secondes, de préférence une polymérisation inférieure à 1 minute, sans occasionner de brûlure des tissus.

Par ailleurs, la présence combinée d'une résine photopolymérisable, avec les monomères polymérisables utilisés et le photoamorceur, apporte au collage obtenu des propriétés de flexibilité, une bonne stabilité dans le temps sous l'action des liquides physiologiques (*e.g.* sang, exsudats, transpiration), empêche l'accumulation de transpiration ou d'exsudats et confère une bonne résistance à l'eau.

Le procédé selon l'invention est préférentiellement non-invasif. Le terme « non-invasif » entend signifier que le procédé selon l'invention ne comprend aucune étape chirurgicale consistant à accéder au tissu à traiter. Ainsi, le procédé selon l'invention est mis en œuvre sur un tissu biologique directement accessible (e.g. la peau) ou préalablement rendu accessible par d'autres méthodes.

Le procédé selon l'invention est préférentiellement un procédé non-invasif pour recouvrir et protéger une lésion cutanée. Le procédé selon l'invention est préférentiellement un procédé non-invasif pour rapprocher les berges d'une plaie cutanée. Par « cutané », on entend désigner une localisation située sur la peau, les lèvres ou la muqueuse buccale.

Alternativement, le procédé selon l'invention est un procédé pour l'adhésion d'un matériau à un tissu biologique, pour l'adhésion de tissus biologiques entre eux, pour l'adhésion d'une colle ou d'une substance à la surface d'un tissu biologique, l'étanchéification chirurgical, pour obturer ou colmater les orifices crées par une suture par fil ou par agrafe ou par une résection tissulaire (hémostase, aérostase, lymphostase par exemple), pour obturer un orifice une incision ou une déchirure dans un tissu biologique, pour arrêter un saignement, pour recouvrir et protéger une plaie, pour renforcer un tissu biologique, pour prévenir la formation de lésions sur un tissu biologique, ou pour fixer et stabiliser un tissu biologique.

### EXEMPLES

La présente invention se comprendra mieux à la lecture des exemples suivants qui illustrent non-limitativement l'invention.

### Exemple 1 : Evaluation de compositions selon l'invention

**But :** Différentes compositions polymérisables selon l'invention ont été préparées et évaluées pour leur utilisation en tant qu'adhésif pour tissus biologiques.

### Compositions :

Des compositions selon l'invention ont été préparées avec les composés suivants, disponibles commercialement :
- photoamorceur : TPO-L (phényl(2,4,6-triméthylbenzoyl)phosphinate d'éthyle) ;
- résine photopolymérisable : résine uréthane acrylate aliphatique Allnex Ebecryl 9907^{®} ; résine époxy acrylate Allnex Ebecryl 3708^{®} (correspondant à une résine bisphénol A époxy diacrylate) ; résine époxy acrylate Genomer 2312^{®} (correspondant à une résine d'acrylate d'huile de soja époxydé) de la société RAHN ;
- monomères polymérisables non-phosphatés : acrylate de *tert*-butyle (tBuA), méthacrylate d'hydroxyéthyle (HEMA), acide acrylique (AA), acrylate de lauryle (LA), acrylate d'isobornyle (IBOA), méthacrylate d'isobornyle (IBOMA), méthacrylate de 3,3,5 triméthyl cyclohexanol (TMCHMA).

Les compositions listées dans la Table 1 ont été préparées par mélange des différents constituants. Dans la Table 1, les proportions des différents constituants sont données en pourcentage en masse par rapport à la masse totale de la composition.

La composition 15 (sans résine photopolymérisable) ne fait pas partie de l'invention et est présentée dans la Table 1 comme composition témoin.

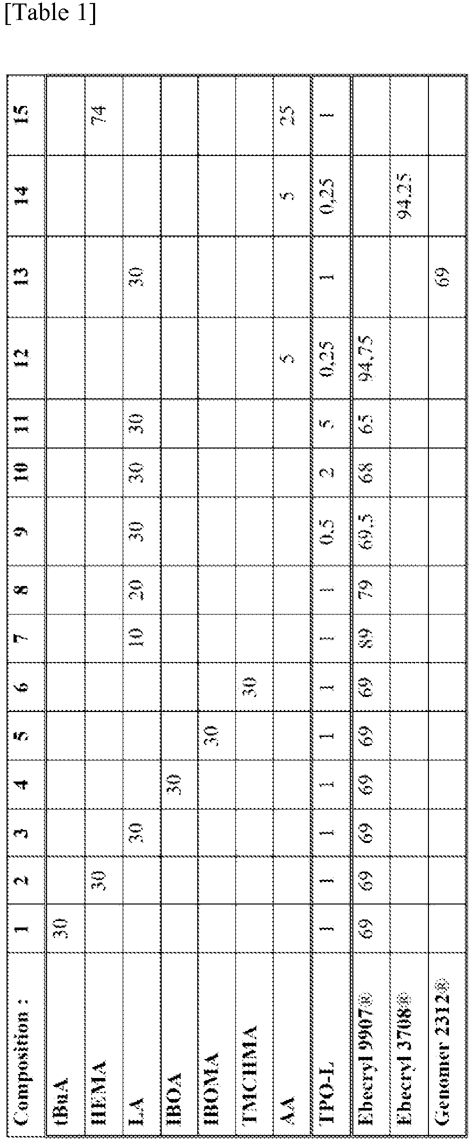

Le spectre d'émission de la source LED UV utilisée pour polymériser les formulations est centré sur 395 nm et utilisée à une irradiance de 20 mW/cm².

### Critères évalués :

- le temps de polymérisation : une photopolymérisation rapide est visée, de préférence d'une durée inférieure à 1 minute, afin notamment de pouvoir contrôler le collage et pour une utilisation facilitée, tout en étant réalisée à des gammes d'irradiance faible à modérée (de préférence de 5 mW/cm² à 250 mW/cm²) qui sont adaptées à une utilisation sur des tissus biologiques (i.e. pas d'émission d'UV C) et accessible avec des sources de rayonnement légères, par opposition aux sources de rayonnement industrielles ;
- le taux de conversion : une conversion finale élevée est visée, de préférence un taux de conversion supérieur à 90%, pour une polymérisation optimisée et éviter la présence d'une trop forte proportion de composés non polymérisés dans l'adhésif final qui pourraient présenter une toxicité ;
- l'exothermie : l'exothermie de la réaction de polymérisation doit être limitée pour le confort du sujet et ne doit pas occasionner de brûlure des tissus ;
- l'adhésion aux tissus et la tenue du collage dans le temps (en particulier la tenue *in vivo*) : l'adhésion et la tenue dans le temps doivent être suffisamment bonnes pour accompagner durablement le processus de cicatrisation du tissu et limiter le nombre de changement de pansement, à l'origine d'irritations ou de lésions du tissu périlésionnel ;
- la résistance à l'eau : une bonne résistance à l'eau (et aux fluides biologique) est recherchée afin de maintenir les propriétés d'adhésion en milieu humide, d'éviter la déformation au contact de l'eau/fluides physiologiques pour maintenir la protection mécanique et l'adhésion au tissu biologique, et de protéger durablement la peau en cas de contact ou d'immersion dans un milieu aqueux (activités ménagères quotidiennes, lavage de main, douche, activités nautiques...) tout en évitant la macération du tissu sous le collage ;
- la flexibilité du réseau formé :le réseau formé doit avoir une flexibilité adaptée à un usage sur tissu biologique mou afin de protéger le tissu de l'extérieur et d'assurer un confort de port (absorption des chocs, résistant et résilient face aux grandes déformations) ;
- la résistance mécanique du réseau formé, incluant la résistance à l'effritement et à la déchirure.

### Méthodologie :

L'évaluation qualitative de l'adhésion *in vivo* est réalisée par une évaluation cutanée qualitative sur la main de testeurs volontaires (N = 1 à 5 tests). Une échelle d'adhésion qualitative a été mise au point (Table 2) prenant en compte les critères d'évaluation de l'adhésion et de la tenue suivants :
- adhésion à t = 0 ;
- ancrage dans la couche superficielle de la peau ;
- tenue dans le temps ; et
- résistance à la sollicitation.

**[Table 2]**

| | |
|---|---|
| 0 | pas d'adhésion à t = 0, aucun ancrage |
| 1 | adhésion à t = 0, très faible ancrage, décollement en quelques minutes, aucune résistance à la sollicitation |
| 2 | adhésion à t = 0, faible ancrage, tenue < 1h, pas de résistance à la sollicitation |
| 3 | adhésion à t = 0, faible ancrage, tenue 2h à 4h, faible résistance à la sollicitation |
| 4 | adhésion à t = 0, faible ancrage, tenue 4h à 8h, faible résistance à la sollicitation |
| 5 | adhésion à t = 0, ancrage moyen, tenue 4h à 8h, résistance à la sollicitation moyenne |
| 6 | adhésion à t = 0, ancrage moyen, tenue 8h à 12h, résistance à la sollicitation moyenne |
| 7 | adhésion à t = 0, ancrage moyen, tenue 12h à 24h, résistance à la sollicitation bonne à moyenne |
| 8 | adhésion à t = 0, bon ancrage, tenue 24h à 36h, bonne résistance à la sollicitation |
| 9 | adhésion à t = 0, bon ancrage, tenue 36h à 48h, très bonne résistance à la sollicitation |
| 10 | adhésion à t = 0, excellent ancrage, tenue > 48h, excellente résistance à la sollicitation |

Le temps de polymérisation et le taux de conversion final sont évalués par suivi cinétique de photopolymérisation en ATR-IR. La disparition des bandes d'absorption IR caractéristiques des acrylates est quantifiée sous rayonnement UV LED 395nm à une irradiance de 17 mW/cm².

L'exothermie de photopolymérisation est évaluée qualitativement *in vivo* par une évaluation cutanée qualitative sur la main de testeurs volontaires. L'échelle de ressenti est décrite ainsi :
- 0: pas de chaleur ressentie ;
- +: exothermie faible ressentie ;
- ++: exothermie modérée ressentie ;
- +++: exothermie élevée ressentie ;
- x: douleur à la polymérisation.

La flexibilité est évaluée qualitativement par deux moyens. D'une part, la résistance à la courbure de films polymères obtenus par polymérisation sous rayonnement LED UV 395 nm pendant 1 min à une irradiance de 17 mW/cm², est évaluée qualitativement. D'autre part, la flexibilité est évaluée qualitativement *in vivo* via une appréciation du confort de port sur la main de testeurs volontaires. L'échelle de flexibilité suivante est utilisée :
- 0: film cassant vitreux, inconfortable, peut causer une irritation sur les bords du collage ;
- +: film déformable mais rigide, inconfortable ;
- ++: film flexible, confortable ;
- +++: film très flexible, confortable, suit parfaitement les mouvements du tissu biologique.

La résistance mécanique est évaluée qualitativement par deux moyens. D'une part, des films polymères obtenus par polymérisation sous rayonnement LED UV 395 nm pendant 1 min à une irradiance de 17 mW/cm², sont soumis à une sollicitation manuelle de déchirure. D'autre part, la résistance à l'effritement est évaluée qualitativement par cisaillement manuel de la surface et des bords de la plaque. L'échelle de résistance mécanique suivante est utilisée :
- 0: texture hydrogel, s'effrite et se déchire facilement sous la contrainte ;
- +: fragile et sensible à la déchirure ;
- ++: sensibilité moyenne à la déchirure ;
- +++: résistant à la déchirure ;
- ++++: très résistant à la déchirure.

La sensibilité à l'eau est évaluée qualitativement par deux moyens. Dans un premier temps, des films polymères obtenus par polymérisation sous rayonnement LED UV 395 nm pendant 1 min à une irradiance de 17 mW/cm², sont immergés dans l'eau à 40°C pendant 24h à 72h et des observations macroscopiques (blanchiment, déformation, assouplissement, fragilisation) sont recueillies. Dans un deuxième temps, la sensibilité à l'eau est évaluée qualitativement par un test de lavage de main de testeurs volontaires. L'échelle de sensibilité à l'eau suivante est utilisée :
- 0: absence d'évolution ;
- +: gonflement faible à modéré ;
- ++: blanchiment/gonflement élevé ;
- +++: blanchiment/gonflement élevé/déformation/perte de résistance mécanique.
- ++++: Altération physique totale (destruction de l'échantillon)

### Résultats

Les résultats obtenus pour les différentes compositions testées sont rapportés dans la Table 3.

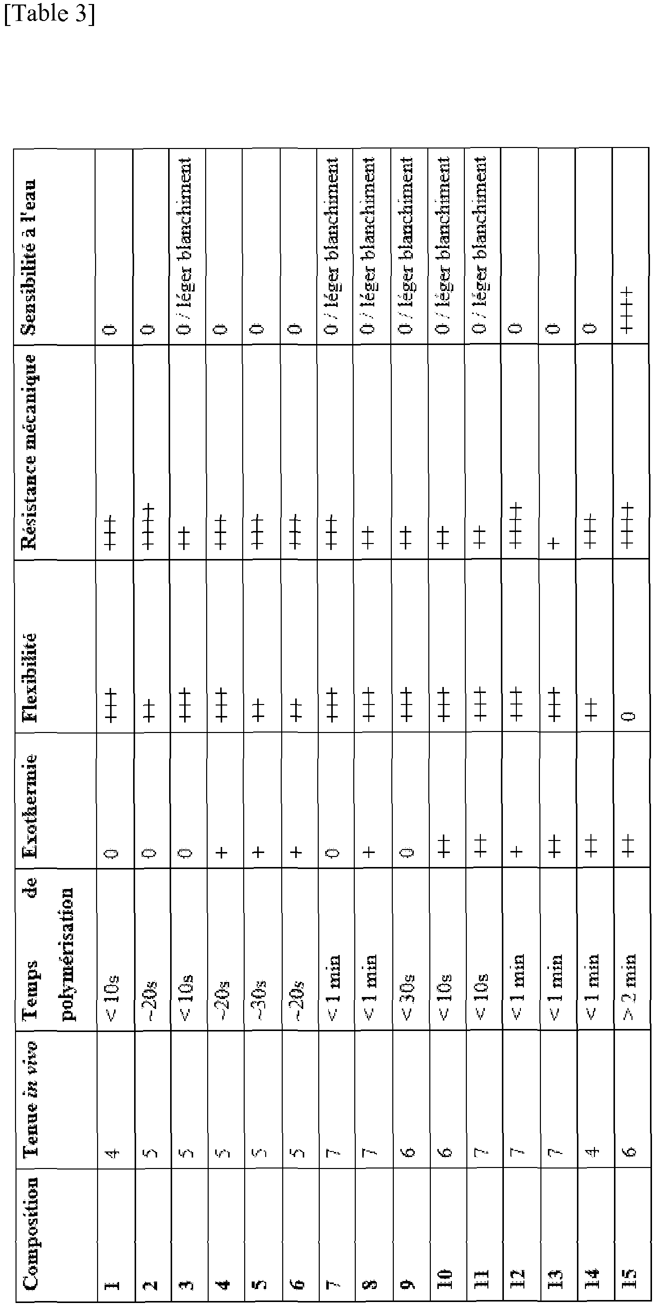

Les taux de conversion obtenus pour toutes les compositions de l'invention testées ci-dessus sont supérieurs à 90%.

Les compositions de l'invention présentent les propriétés recherchées d'adhésion à un tissu biologique ainsi que les propriétés recherchées de flexibilité et de conformabilité adaptées aux tissus biologiques non minéralisés. Par ailleurs, les compositions de l'invention permettent d'obtenir des collages ayant une bonne stabilité dans le temps.

En revanche, les résultats montrent qu'une composition ne comprenant pas de résine photopolymérisable, ne permet pas d'obtenir à la fois de bonnes propriétés d'adhésion et de flexibilité. De plus, le film polymère issu de cette composition est très sensible à l'eau. En effet, le film polymère issu de la composition 15 a été fortement altéré et son immersion dans l'eau à 40°C pendant 24h à 72h a conduit à la destruction de ce film polymère.

## Revendications

1. Composition polymérisable, destinée à être utilisée en tant qu'adhésif pour tissus biologiques non minéralisés, comprenant :
- de 5 % à 60 % en masse, par rapport à la masse totale de la composition, d'un monomère polymérisable comprenant une fonction acrylate, méthacrylate, acrylamide ou méthacrylamide et ne comprenant pas de fonction phosphate ou phosphonate ;
- de 0,1 % à 5 % en masse d'un photoamorceur ; et
- une résine photopolymérisable ;
ladite composition ne comprenant pas de monomère comprenant une fonction phosphate ou phosphonate d'une part et une fonction acrylate, méthacrylate, acrylamide ou méthacrylamide d'autre part.

2. Composition selon la revendication **1,** dans laquelle le monomère polymérisable est choisi parmi : acide acrylique (AA), acrylate de *tert-butyl* (tBuA), méthacrylate de 2-hydroxyéthyl (HEMA), acide méthacrylique (MA), acrylate de lauryle (LA), méthacrylate de lauryle (LMA), méthacrylate de 2-éthoxyéthyle (2EEMA), acrylate de di(éthylène glycol) éthyle éther (DEGEA), acrylate de 2-phényloxyéthyle (2PEA), méthacrylate de 2-éthylhexyle (2EHMA), acrylate de n-butyle (nBuA), acrylate d'isobornyle (IBOA), méthacrylate d'isobornyle (IBOMA), cyclic triméthylolpropane formal acrylate (CTFA), méthacrylate de 3,3,5 triméthyl cyclohexanol, méthacrylate de tert-butyl (tBuMA), acrylate de méthyle (MeA), méthacrylate de méthyle (MMA), acrylate de 2-éthylhexyle (2EHA), acrylate de 2-(diméthylamino)éthyle (DAEA), sel potassique d'acrylate de 3-sulfopropyle (SAPS), acide 3,3-diméthylacrylique (DAA), acide crotonique (CA), méthacrylate de triéthylène glycol méthyle éther (TEGMEMA), méthacrylate de 2-phényloxyéthyle (2PEMA), acrylate de 2-hydroxyéthyle (HEA), méthacrylate de 3-(triméthoxysilyl)propyle, acrylate d'éthyle (EtA), méthacrylate de cyclohexyle, acrylate de 3-hydroxypropyle, acrylate d'alpha-bromoéthyle, acrylate d'alpha-chloroéthyle, méthacrylate de chlorométhyle, méthacrylate de 2-bromoéthyle, méthacrylate de 2-naphtyle, acrylate de paratolyle, méthacrylate de parachlorophényle, acrylate de metabromophényle, acrylate de 2,4,6-tribromophényle, méthacrylate de parachlorobenzyle, méthacrylate de metaméthoxybenzyle, acrylate de paraéthylbenzyle, diméthacrylate de 1,6-hexanediol, diacrylate de neopentylglycol, diméthacrylate de thiodiéthylène-glycol, diacrylate de bisphénol A éthoxyle, diméthacrylate de bisphénol A éthoxyle, triacrylate de pentaérythritol, triacrylate de glycéryle, pentaacrylate de dipentaérythritol, triacrylate de triméthylolpropane, triméthacrylate de l'isocyanurate de tris(2-hydroxyéthyle), triacrylate de triméthylolpropane polyoxyéthylène, acrylate d'uréthane, methacrylate d'uréthane, sulfure de bis(4-methacryloylthiophényle), acrylate d'éthylèneglycol, acrylate de polyéthylèneglycol, méthacrylate d'éthylèneglycol, méthacrylate de polyéthylèneglycol, diacrylate d'éthylèneglycol, diacrylate de polyéthylèneglycol, diméthacrylate d'éthylèneglycol, diméthacrylate de polyéthylèneglycol, et leurs mélanges.

3. Composition selon la revendication **1** ou **2,** dans laquelle le monomère polymérisable est choisi parmi : acide acrylique (AA), acrylate de tert-butyl (tBuA), méthacrylate de 2-hydroxyéthyl (HEMA), acide méthacrylique (MA), acrylate de lauryle (LA), méthacrylate de lauryle (LMA), méthacrylate de 2-éthoxyéthyle (2EEMA), acrylate de di(éthylène glycol) éthyle éther (DEGEA), acrylate de 2-phényloxyéthyle (2PEA), méthacrylate de 2-éthylhexyle (2EHMA), acrylate de *n*-butyle (nBuA), acrylate d'isobornyle (IBOA), méthacrylate d'isobornyle (IBOMA), cyclic triméthylolpropane formal acrylate (CTFA), méthacrylate de 3,3,5 triméthyl cyclohexanol, méthacrylate de *tert-butyl* (tBuMA), acrylate de méthyle (MeA), méthacrylate de méthyle (MMA), acrylate de 2-éthylhexyle (2EHA), acrylate de 2-(diméthylamino)éthyle (DAEA), sel potassique d'acrylate de 3-sulfopropyle (SAPS), acide 3,3-diméthylacrylique (DAA), acide crotonique (CA), méthacrylate de triéthylène glycol méthyle éther (TEGMEMA), méthacrylate de 2-phényloxyéthyle (2PEMA), acrylate de 2-hydroxyéthyle (HEA), méthacrylate de 3-(triméthoxysilyl)propyle, et leurs mélanges.

4. Composition selon l'une quelconque des revendications **1** à **3,** dans laquelle le monomère polymérisable est à une concentration allant de 10 % à 40 % en masse par rapport à la masse totale de la composition.

5. Composition selon l'une quelconque des revendications **1** à **4,** dans laquelle le photoamorceur est choisi parmi l'oxyde de 2,4,6-triméthylbenzoyl-phénylphosphinate (TPO-L), la camphorquinone, la 4,4'-bis(diéthylamino)benzophénone, la 4,4'-bis(diéthylamino)benzophénone associée à la N-phénylglycine (NPG), à l'éthyl-4- (diméthylamino)benzoate (EDB), à la N-diisopropyléthylamine (DIPEAN) ou au 4-(diméthylamino)benzonitrile (DMABN), l'oxyde de biacylphosphine (BAPO), le bis(.eta.5-2,4-cylcopentadièn-1-yl)-bis(2,6-difluoro-3-(1H-pyrrol-1-yl)-phényl) titanium (Irgacure 784), la 1-[4-(2-hydroxyéthoxy)-phényl]-2-hydroxy-2-méthyl-1-propane-1-one (Irgacure 2959), l'oxyde de 2,4,6-triméthylbenzoyldiphénylphosphine (TPO), la 2,2-diméthoxyphényl-2-acétophénone (DMPA), et leurs mélange.

6. Composition selon l'une quelconque des revendications **1** à **5,** dans laquelle le photoamorceur est à une concentration allant de 0,1 % à 2 % en masse par rapport à la masse totale de la composition.

7. Composition selon l'une quelconque des revendications **1** à **6,** dans laquelle la résine photopolymérisable est choisie parmi : les résines uréthane acrylate, les résines uréthane méthacrylate, les résines époxy acrylate, et leurs mélanges ; de préférence la résine photopolymérisable est choisie parmi les résines uréthane acrylate, les résines uréthane méthacrylate, les résines époxy acrylate difonctionnelle et leurs mélanges; plus préférentiellement la résine photopolymérisable est choisie parmi : une résine uréthane acrylate, une résine uréthane méthacrylate et leurs mélanges; encore plus préférentiellement, la résine photopolymérisable est sélectionnée parmi les résines uréthanes acrylates aliphatiques, les résines uréthanes acrylates hydrophobes, les résines uréthanes acrylates aromatiques, les résines polyéthers uréthanes acrylates, les résines uréthanes méthacrylates aliphatiques, les résines uréthanes méthacrylates hydrophobes, les résines uréthanes méthacrylates aromatiques, les résines polyéthers uréthanes méthacrylates, et leurs mélanges.

8. Composition selon l'une quelconque des revendications **1** à **7,** dans laquelle la résine photopolymérisable est à une concentration allant de 10% à 94,9 % en masse par rapport à la masse totale de la composition.

9. Composition selon l'une quelconque des revendications **1** à **8,** pour son utilisation pour l'adhésion de tissus biologiques non minéralisés entre eux ; pour l'adhésion d'un matériau à un tissu biologique non minéralisé ; pour l'adhésion d'une colle ou d'une substance à la surface d'un tissu biologique non minéralisé ; en tant qu'étanchéificateur chirurgical sur un tissu biologique non minéralisé ; pour obturer ou colmater les orifices crées par une suture par fil ou par agrafe ou par une résection tissulaire dans un tissu biologique non minéralisé ; pour obturer un orifice, une incision ou une déchirure dans un tissu biologique non minéralisé ; en tant qu'hémostatique pour arrêter un saignement sur un tissu biologique non minéralisé , en tant que pansement sur un tissu biologique non minéralisé pour recouvrir et protéger une plaie ; pour renforcer un tissu biologique non minéralisé ; pour prévenir la formation de lésions sur un tissu biologique non minéralisé, pour fixer et stabiliser un tissu biologique non minéralisé ; et/ou pour le traitement des lésions cutanées.

10. Composition pour son utilisation selon la revendication **9,** comprenant la mise en contact de la composition avec le tissu biologique non minéralisé à traiter, de préférence par étalement ; et la photopolymérisation de ladite composition.

11. Procédé de préparation d'une composition polymérisable destinée à être utilisée en tant qu'adhésif pour tissus biologiques non minéralisés, comprenant une étape de mélange :
- d'au moins un monomère polymérisable comprenant une fonction acrylate, méthacrylate, acrylamide ou méthacrylamide et ne comprenant pas de fonction phosphate ou phosphonate;
- d'au moins un photoamorceur ; et
- d'au moins une résine photopolymérisable ;
ledit mélange ne comprenant pas de monomère comprenant une fonction phosphate ou phosphonate d'une part et une fonction acrylate, méthacrylate, acrylamide ou méthacrylamide d'autre part.

12. Procédé selon la revendication **11,** dans lequel la masse du monomère polymérisable varie de 5 % à 60% en masse, par rapport à la masse totale dudit mélange; et la masse du photoamorceur varie de 0,1% à 5% en masse, par rapport à la masse totale dudit mélange.

13. Kit comprenant un dispositif renfermant la composition polymérisable selon l'une quelconque des revendications **1** à **8.**

14. Kit selon la revendication **13,** comprenant en outre une source de rayonnement.

## Patentansprüche

1. Polymerisierbare Zusammensetzung, die zur Verwendung als Haftstoff für nicht mineralisiertes biologisches Gewebe bestimmt ist, umfassend:
- 5 bis 60 Massenprozent, bezogen auf die Gesamtmasse der Zusammensetzung, eines polymerisierbaren Monomers, das eine Acrylat-, Methacrylat-, Acrylamid- oder Methacrylamidfunktion umfasst und keine Phosphat- oder Phosphonatfunktion umfasst;
- 0,1 bis 5 Massenprozent eines Photoprimers; und
- lichthärtendes Harz;
wobei die Zusammensetzung kein Monomer umfasst, das einerseits eine Phosphatoder Phosphonatfunktion und andererseits eine Acrylat-, Methacrylat-, Acrylamidoder Methacrylamidfunktion umfasst.

2. Zusammensetzung nach Anspruch **1,** wobei das polymerisierbare Monomer aus Folgendem ausgewählt ist: Acrylsäure (AA), t*ert*-Butylacrylat (tBuA), 2-Hydroxyethylmethacrylat (HEMA), Methacrylsäure (MA), Laurylacrylat (LA), Laurylmethacrylat (LMA), 2-Ethoxyethylmethacrylat (2EEMA), Di(ethylenglykol)ethyletheracrylat (DEGEA), 2-Phenyloxyethylacrylat (2PEA), 2-Ethylhexylmethacrylat (2EHMA), *n*-Butylacrylat (nBuA), Isobornylacrylat (IBOA), Isobornylmethacrylat (IBOMA), zyklischem Trimethylolpropanformalacrylat (CTFA), 3,3,5-Trimethylcyclohexanolmethacrylat, *tert*-Butylmethacrylat (tBuMA), Methylacrylat (MeA), Methylmethacrylat (MMA), 2-Ethylhexylacrylat (2EHA), 2-(Dimethylamino)ethylacrylat (DAEA), 3-Sulfopropylacrylat-Kaliumsalz (SAPS), 3,3-Dimethylacrylsäure (DAA), Crotonsäure (CA), Triethylenglykolmethylethermethacrylat (TEGMEMA), 2-Phenyloxyethylmethacrylat (2PEMA), 2-Hydroxyethylacrylat (HEA), 3-(Trimethoxysilyl)propylmethacrylat, Ethylacrylat (EtA), Cyclohexylmethacrylat, 3-Hydroxypropylacrylat, Alphabromethylacrylat, Alphachlorethylacrylat, Chlormethylmethacrylat, 2-Bromethylmethacrylat, 2-Naphtylmethacrylat, Paratolylacrylat, Parachlorphenylmethacrylat, Metabromphenylacrylat, 2,4,6-Tribromophenylacrylat, Parachlorbenzylmethacrylat, Methamethoxybenzylmethacrylat, Paraethylbenzylacrylat, 1,6-Hexandioldimethacrylat, Neopentylglykoldiacrylat, Thiodiethylenglykoldimethacrylat, Bisphenol-A-Ethoxyldiacrylat, Bisphenol-A-Ethoxyldimethacrylat, Pentaerythritoltriacrylat, Glyceryltriacrylat, Dipentaerythritolpentaacrylat, Trimethylolpropantriacrylat, Tris(2-hydroxyethylisocyanurattrimethacrylat, Polyoxyethylentrimethylolpropantriacrylat, Urethanacrylat, Urethanmethacrylat, Bis(4-methacryloylthiophenyl)sulfid, Ethylenglykolacrylat, Polyethylenglykolacrylat, Ethylenglykolmethacrylat, Polyethylenglykolmethacrylat, Ethylenglykoldiacrylat, Polyethylenglykoldiacrylat, Ethylenglykoldimethacrylat, Polyethylenglykoldimethacrylat und Mischungen davon.

3. Zusammensetzung nach Anspruch **1** oder **2,** wobei das polymerisierbare Monomer aus Folgendem ausgewählt ist: Acrylsäure (AA), *tert*-Butylacrylat (tBuA), 2-Hydroxyethylmethacrylat (HEMA), Methacrylsäure (MA), Laurylacrylat (LA), Laurylmethacrylat (LMA), 2-Ethoxyethylmethacrylat (2EEMA), Di(ethylenglykol)ethyletheracrylat (DEGEA), 2-Phenyloxyethylacrylat (2PEA), 2-Ethylhexylmethacrylat (2EHMA), n-Butylacrylat (nBuA), Isobornylacrylat (IBOA), Isobornylmethacrylat (IBOMA), zyklischem Trimethylolpropanformiacrylat (CTFA), 3,3,5-Trimethylcyclohexanolmethacrylat, t*ert*-Butylmethacrylat (tBuMA), Methylacrylat (MeA), Methylmethacrylat (MMA), 2-Ethylhexylacrylat (2EHA), 2-(Dimethylamino)ethylacrylat (DAEA), 3-Sulfopropylacrylat-Kaliumsalz (SAPS), 3,3-Dimethylacrylsäure (DAA), Crotonsäure (CA), Triethylenglykolmethylethermethacrylat (TEGMEMA), 2-Phenyloxyethylmethacrylat (2PEMA), 2-Hydroxyethylacrylat (HEA), 3-(Trimethoxysilyl)propylmethacrylat und Mischungen davon.

4. Zusammensetzung nach einem der Ansprüche **1** bis **3,** wobei das polymerisierbare Monomer in einer Konzentration von 10 bis 40 Massenprozent, bezogen auf die Gesamtmasse der Zusammensetzung, vorliegt.

5. Zusammensetzung nach einem der Ansprüche **1** bis **4,** wobei der Photoprimer ausgewählt ist aus 2,4,6-Trimethylbenzoyl-phenylphosphinat (TPO-L)-Oxid, Camphorquinon, 4,4'-bis(Diethylamino)benzophenon, 4,4'-bis(Diethylamino)benzophenon in Verbindung mit N-Phenylglycin (NPG), Ethyl-4-(Dimethylamino)benzoat (EDB), N-Diisopropylethylamin (DIPEAN) oder 4-(Dimethylamino)benzonitril (DMABN), Biacylphosphinoxid (BAPO), Bis(.eta.5-2,4-cylcopentadien-1-yl)-bis(2,6-difluoro-3-(1H-pyrrol-1-yl)-phenyl)titanium (Irgacure 784), 1-[4-(2-hydroxyethoxy)-phenyl]-2-hydroxy-2-methyl-1-propan-1-on (Irgacure 2959), 2,4,6-trimethylbenzoyldiphenylphosphinoxid (TPO), 2,2-dimethoxyphenyl-2-acetophenon (DMPA) und Mischungen davon.

6. Zusammensetzung nach einem der Ansprüche **1** bis **5,** wobei der Photoprimer in einer Konzentration von 0,1 bis 2 Massenprozent, bezogen auf die Gesamtmasse der Zusammensetzung, vorliegt.

7. Zusammensetzung nach einem der Ansprüche **1** bis **6,** wobei das lichthärtende Harz aus Folgendem ausgewählt ist: Urethanacrylatharzen, Urethanmethacrylatharzen, Epoxidacrylatharzen und Mischungen davon; vorzugsweise ist das lichthärtende Harz ausgewählt aus Urethanacrylatharzen, Urethanmethacrylatharzen, difunktionalen Epoxidacrylatharzen und Mischungen davon; bevorzugter ist das lichthärtende Harz ausgewählt aus: einem Urethanacrylatharz, einem Urethanmethacrylatharz und Mischungen davon; noch bevorzugter ist das lichthärtende Harz ausgewählt aus aliphatischen Urethanacrylatharzen, hydrophoben Urethanacrylatharzen, aromatischen Urethanacrylatharzen, Polyetherurethanacrylatharzen, aliphatischen Urethanmethacrylaten, hydrophoben Urethanmethacrylaten, aromatischen Urethanmethacrylaten, Methacrylat-Polyetherurethanharzen und Mischungen davon.

8. Zusammensetzung nach einem der Ansprüche **1** bis **7,** wobei das lichthärtende Harz in einer Konzentration von 10 bis 94,9 Massenprozent, bezogen auf die Gesamtmasse der Zusammensetzung, vorliegt.

9. Zusammensetzung nach einem der Ansprüche **1** bis **8,** zur Verwendung zur Haftung von nicht mineralisiertem biologischem Gewebe untereinander; zur Haftung eines Materials an nicht mineralisiertem biologischem Gewebe; zur Haftung eines Klebstoffs oder einer Substanz an der Oberfläche eines nicht mineralisierten biologischen Gewebes; als chirurgisches Dichtmittel auf nicht mineralisiertem biologischem Gewebe; zum Verschließen oder Verschließen von Löchern, die durch Naht oder Klammernaht oder durch Geweberesektion in nicht mineralisiertem biologischem Gewebe entstanden sind; zum Verschließen von Löchern, Inzisionen oder Rissen in nicht mineralisiertem biologischem Gewebe; als Hämostatikum zum Stoppen von Blutungen in nicht mineralisiertem biologischem Gewebe, als Verband in nicht mineralisiertem biologischem Gewebe zur Abdeckung und zum Schutz einer Wunde; zur Verstärkung von nicht mineralisiertem biologischem Gewebe; zur Verhinderung der Bildung von Schäden an nicht-mineralisiertem biologischem Gewebe, zur Fixierung und Stabilisierung von nicht-mineralisiertem biologischem Gewebe und/oder zur Behandlung von Hautschäden.

10. Zusammensetzung zur Verwendung nach Anspruch **9,** umfassend das Inkontaktbringen der Zusammensetzung mit dem zu behandelnden nicht mineralisierten biologischen Gewebe, vorzugsweise durch Verteilen; und das Lichtaushärten der Zusammensetzung.

11. Verfahren zur Herstellung einer polymerisierbaren Zusammensetzung, die zur Verwendung als Haftstoff für nicht mineralisiertes biologisches Gewebe bestimmt ist, umfassend einen Mischschritt:
- mindestens eines polymerisierbaren Monomers, das eine Acrylat-, Methacrylat-, Acrylamid- oder Methacrylamidfunktion umfasst und keine Phosphat- oder Phosphonatfunktion umfasst;
- mindestens eines Photoprimers; und
- mindestens eines lichthärtenden Harzes;
wobei die Mischung kein Monomer umfasst, das einerseits eine Phosphat- oder Phosphonatfunktion und andererseits eine Acrylat-, Methacrylat-, Acrylamid- oder Methacrylamidfunktion umfasst.

12. Verfahren nach Anspruch **11,** wobei die Masse des polymerisierbaren Monomers, bezogen auf die Gesamtmasse der Mischung, von 5 bis 60 Massenprozent variiert; und die Masse des Photoprimers, bezogen auf die Gesamtmasse der Mischung, von 0,1 bis 5 Massenprozent variiert.

13. Set, das eine Vorrichtung umfasst, die die polymerisierbare Zusammensetzung nach einem der Ansprüche **1** bis **8** umfasst.

14. Set nach Anspruch **13,** ferner umfassend eine Strahlungsquelle.

## Claims

1. A polymerizable composition, intended for use as an adhesive for non-mineralized biological tissues, comprising:
- from 5% to 60% by mass, relative to the total mass of the composition, of a polymerizable monomer comprising an acrylate, methacrylate, acrylamide, or methacrylamide function and not comprising a phosphate or phosphonate function;
- from 0.1% to 5% by mass of a photoinitiator; and
- a photopolymerizable resin;
said composition not comprising a monomer comprising a phosphate or phosphonate function on the one hand and an acrylate, methacrylate, acrylamide, or methacrylamide function on the other hand.

2. The composition according to claim 1, wherein the polymerizable monomer is selected from: acrylic acid (AA), *tert-butyl* acrylate (tBuA), 2-hydroxyethyl methacrylate (HEMA), methacrylic acid (MA), lauryl acrylate (LA), lauryl methacrylate (LMA), 2-ethoxyethyl methacrylate (2EEMA), di(ethylene glycol) ethyl ether acrylate (DEGEA), 2-phenyloxyethyl acrylate (2PEA), 2-ethylhexyl methacrylate (2EHMA), *n*-butyl acrylate (nBuA), isobornyl acrylate (IBOA), isobornyl methacrylate (IBOMA), cyclic trimethylolpropane formal acrylate (CTFA), 3,3,5 trimethyl cyclohexanol methacrylate, *tert-butyl* methacrylate (tBuMA), methyl acrylate (MeA), methyl methacrylate (MMA), 2-ethylhexyl acrylate (2EHA), 2-(dimethylamino)ethyl acrylate (DAEA), 3-sulfopropyl acrylate potassium salt (SAPS), 3,3-dimethylacrylic acid (DAA), crotonic acid (CA), triethylene glycol methyl ether methacrylate (TEGMEMA), 2-phenyloxyethyl methacrylate (2PEMA), 2-hydroxyethyl acrylate (HEA), 3-(trimethoxysilyl)propyl methacrylate, ethyl acrylate (EtA), cyclohexyl methacrylate, 3-hydroxypropyl acrylate, alpha-bromoethyl acrylate, alphachloroethyl acrylate, chloromethyl methacrylate, 2-bromoethyl methacrylate, 2-naphthyl methacrylate, paratolyl acrylate, parachlorophenyl methacrylate, metabromophenyl acrylate, 2,4,6-tribromophenyl acrylate, parachlorobenzyl methacrylate, metamethoxybenzyl methacrylate, paraethylbenzyl acrylate, 1,6-hexanediol dimethacrylate, neopentyl glycol diacrylate, thiodiethylene glycol dimethacrylate, bisphenol A ethoxylate diacrylate, bisphenol A ethoxylate dimethacrylate, pentaerythritol triacrylate, glyceryl triacrylate, dipentaerythritol pentaacrylate, trimethylolpropane triacrylate, tris(2-hydroxyethyl) isocyanurate trimethacrylate, trimethylolpropane polyoxyethylene triacrylate, urethane acrylate, urethane methacrylate, bis(4-methacryloylthiophenyl) sulfide, ethylene glycol acrylate, polyethylene glycol acrylate, ethylene glycol methacrylate, polyethylene glycol methacrylate, ethylene glycol diacrylate, polyethylene glycol diacrylate, ethylene glycol dimethacrylate, polyethylene glycol dimethacrylate, and mixtures thereof.

3. The composition according to claim 1 or 2, wherein the polymerizable monomer is selected from: acrylic acid (AA), *tert-butyl* acrylate (tBuA), 2-hydroxyethyl methacrylate (HEMA), methacrylic acid (MA), lauryl acrylate (LA), lauryl methacrylate (LMA), 2-ethoxyethyl methacrylate (2EEMA), di(ethylene glycol) ethyl ether acrylate (DEGEA), 2-phenyloxyethyl acrylate (2PEA), 2-ethylhexyl methacrylate (2EHMA), n-butyl acrylate (nBuA), isobornyl acrylate (IBOA), isobornyl methacrylate (IBOMA), cyclic trimethylolpropane formal acrylate (CTFA), 3,3,5 trimethyl cyclohexanol methacrylate, *tert-butyl* methacrylate (tBuMA), methyl acrylate (MeA), methyl methacrylate (MMA), 2-ethylhexyl acrylate (2EHA), 2-(dimethylamino)ethyl acrylate (DAEA), potassium salt of 3-sulfopropyl acrylate (SAPS), 3,3-dimethylacrylic acid (DAA), crotonic acid (CA), triethylene glycol methyl ether methacrylate (TEGMEMA), 2-phenyloxyethyl methacrylate (2PEMA), 2-hydroxyethyl acrylate (HEA), 3-(trimethoxysilyl)propyl methacrylate, and mixtures thereof.

4. The composition according to any one of claims 1 to 3, wherein the polymerizable monomer is at a concentration ranging from 10% to 40% by mass relative to the total mass of the composition.

5. The composition according to any one of claims 1 to 4, wherein the photoinitiator is selected from 2,4,6-trimethylbenzoyl-phenylphosphinic oxide (TPO-L), camphorquinone, 4,4'-bis(diethylamino)benzophenone, 4,4'-bis(diethylamino)benzophenone associated with N-phenylglycine (NPG), ethyl-4-(dimethylamino)benzoate (EDB), N-diisopropylethylamine (DIPEAN) or 4-(dimethylamino)benzonitrile (DMABN), biacylphosphine oxide (BAPO), bis(.eta.5-2,4-cyclopentadien-1-yl)-bis(2,6-difluoro-3-(1H-pyrrol-1-yl)-phenyl) titanium (Irgacure 784), 1-[4-(2-hydroxyethoxy)-phenyl]-2-hydroxy-2-methyl-1-propane-1-one (Irgacure 2959), 2,4,6-trimethylbenzoyldiphenylphosphine oxide (TPO), 2,2-dimethoxyphenyl-2-acetophenone (DMPA), and mixtures thereof.

6. The composition according to any one of claims 1 to 5, wherein the photoinitiator is at a concentration ranging from 0.1% to 2% by mass relative to the total mass of the composition.

7. The composition according to any one of claims 1 to 6, wherein the photopolymerizable resin is selected from: urethane acrylate resins, urethane methacrylate resins, epoxy acrylate resins, and mixtures thereof; preferably the photopolymerizable resin is selected from urethane acrylate resins, urethane methacrylate resins, difunctional epoxy acrylate resins and mixtures thereof; more preferably the photopolymerizable resin is selected from: a urethane acrylate resin, a urethane methacrylate resin and mixtures thereof; even more preferably the photopolymerizable resin is selected from aliphatic urethane acrylate resins, hydrophobic urethane acrylate resins, aromatic urethane acrylate resins, polyether urethane acrylate resins, aliphatic urethane methacrylate resins, hydrophobic urethane methacrylate resins, aromatic urethane methacrylate resins, polyether urethane methacrylate resins and mixtures thereof.

8. The composition according to any one of claims 1 to 7 wherein the photopolymerizable resin is at a concentration ranging from 10% to 94.9% by mass relative to the total mass of the composition.

9. The composition according to any one of claims 1 to 8 for use in adhering non-mineralized biological tissues together; for adhering a material to a non-mineralized biological tissue; for adhering a glue or substance to the surface of a non-mineralized biological tissue; as a surgical sealant on a non-mineralized biological tissue; for sealing or plugging orifices created by suturing with thread or with staples or by tissue resection in a non-mineralized biological tissue; for sealing an orifice, an incision or a tear in a non-mineralized biological tissue; as a hemostatic agent to stop bleeding on a non-mineralized biological tissue; as a dressing on a non-mineralized biological tissue to cover and protect a wound; for reinforcing a non-mineralized biological tissue; for preventing the formation of lesions on a non-mineralized biological tissue; for fixing and stabilizing a non-mineralized biological tissue; and/or for treating skin lesions.

10. The composition for use according to claim 9 comprising contacting the composition with the non-mineralized biological tissue to be treated preferably by spreading; and photopolymerizing said composition.

11. A method for preparing a polymerizable composition intended for use as an adhesive for non-mineralized biological tissues comprising a step of mixing:
- at least one polymerizable monomer comprising an acrylate, methacrylate, acrylamide or methacrylamide function and not comprising a phosphate or phosphonate function;
- at least one photoinitiator; and
- at least one photopolymerizable resin;
said mixture not comprising a monomer comprising a phosphate or phosphonate function on the one hand and an acrylate, methacrylate, acrylamide or methacrylamide function on the other hand.

12. The method according to claim 11 wherein the mass of the polymerizable monomer ranges from 5% to 60% by mass relative to the total mass of said mixture; and the mass of the photoinitiator ranges from 0.1% to 5% by mass relative to the total mass of said mixture.

13. A kit comprising a device containing the polymerizable composition according to any one of claims 1 to 8.

14. The kit according to claim 13 further comprising a radiation source.
